# EUROPÄISCHE PATENTSCHRIFT

(11) **EP 1 735 015 B1**
(45) Veröffentlichungstag und Bekanntmachung des Hinweises auf die Patenterteilung: **09.02.2011**
(21) Anmeldenummer: 05737403.5
(22) Anmeldetag: 12.04.2005
(51) Int. Cl.: A61L 2/26, A61B 19/02

(54) **STERILBEHÄLTER**
STERILE CONTAINER
CONTENANT STERILE

(30) Priorität: 16.04.2004 DE 102004020804
(43) Veröffentlichungstag der Anmeldung: 27.12.2006
(73) Patentinhaber: Aesculap AG, 78532 Tuttlingen (DE)
(72) Erfinder: OERTMANN, Friedrich-Wilhelm, 78532 Tuttlingen (DE)
(74) Vertreter: Hoeger, Stellrecht & Partner Patentanwälte
(86) Internationale Anmeldenummer: PCT/EP2005/003843
(87) Internationale Veröffentlichungsnummer: WO 2005/105161

(56) Entgegenhaltungen:
- GB-A- 1 074 275
- US-A- 5 019 345
- US-A- 5 176 884
- US-A1- 2004 256 268

## Beschreibung

Die Erfindung betrifft einen Sterilbehälter, insbesondere zur Aufnahme und sterilen Aufbewahrung von chirurgischem Besteck oder chirurgischern Material, mit einem durch einen Behälterboden und Behälterwände gebildeten Aufnahmeraum, mit einem Deckel zum Verschließen des Aufnahmeraums, mit einer Sterilbarriere, welche dauerhaft einen Sterilströmungspfad zum Herstellen einer Fluidverbindung zwischen dem Aufnahmeraum und einer Umgebung des Sterilbehälters definiert, und mit einem Überdruckströmungspfad, welcher eine Fluidverbindung zwischen dem Aufnahmeraum und der Umgebung des Sterilbehälters definiert, wobei in einer Sterilstellung des Sterilbehälters, in welcher ein Gasaustausch zwischen dem Aufnahmeraum und der Umgebung des Sterilbehälters nur durch den Sterilströmungspfad möglich ist, der Überdruckströmungspfad geschlossen ist und wobei in einer Überdruckstellung des Sterilbehälters, in welcher eine Druckdifferenz zwischen im Aufnahmeraum und der Umgebung des Sterilbehälters herrschenden Drücken eines Mindestdruckdifferenz übersteigt, der Überdruckströmungspfad mindestens teilweise geöffnet ist.

Sterilbehälter der eingangs beschriebenen Art mit Sterilbarrieren werden verwendet, um einen Fluidaustausch, das heißt einen Austausch von Gasen, Flüssigkeiten oder Gas-Flüssigkeits-Gemischen, beispielsweise Luft, insbesondere während einer Lagerung des Sterilbehälters, zwischen der Umgebung des Sterilbehälters und dem Aufnahmeraum zu ermöglichen. Derartige Behälter sind beispielsweise aus der US 2004/0256268 A1 und der US 5,176,884 bekannt. Während einer Sterilisation des Sterilbehälters können sich große Druckdifferenzen zwischen der Umgebung und dem Aufnahmeraum des Sterilbehälters einstellen, welche zu einer Beschädigung des Sterilbehälters führen können, beispielsweise durch Zusammendrücken oder Aufblasen des Sterilbehälters aufgrund der wirkenden Druckkräfte. Zur Vermeidung von Beschädigungen werden bei Überschreitung einer Mindestdruckdifferenz zusätzliche Nebenstrompfade geöffnet, die einen hohen Luftmassenaustausch in kurzer Zeit gestatten, welcher über den Sterilströmungspfad nicht möglich wäre. Als Sterilbarrieren sind einerseits Filter aus porösem Material bekannt, durch welche Keime und Bakterien nicht durchtreten können, andererseits speziell geformte Strömungspfade, die zwar einen freien Luftdurchlaß ermöglichen, welcher es prinzipiell auch Bakterien und Keimen gestatten würde, ins Innere des Containers zu dringen, wobei jedoch die aerodynamischen Verhältnisse in diesen speziellen Strömungspfaden so ausgelegt sind, daß es Bereiche gibt, in denen keine Strömung existiert. In diesen strömungsfreien Bereichen lagern sich Bakterien und Keime ab und können folglich nicht in den Aufnahmeraum des Sterilbehälters gelangen.

Grundsätzlich wäre es möglich, ein Überdruckventil am Sterilbehälter vorzusehen, welches im Falle eines Überschreitens der Mindestdruckdifferenz einen Gasaustausch zwischen der Umgebung und dem Aufnahmeraum des Sterilbehälters gestattet. Zu diesem Zweck müßte jedoch eine weitere Öffnung im Sterilbehälter vorgesehen werden, zudem wäre ein solches Überdruckventil auch regelmäßig zu warten.

Filterlose Sterilbehälter, die keinen dauerhaft geöffneten Sterilströmungspfad, sondern lediglich Überdruckventile aufweisen, sind aus der GB 1074275 A sowie der US 5,019,345 bekannt.

Es ist daher Aufgabe der vorliegenden Erfindung, einen Sterilbehälter der eingangs beschriebenen Art so zu verbessern, daß ein Aufbau und eine Wartung des Sterilbehälters besonders einfach werden.

Diese Aufgabe wird bei einem Sterilbehälter der eingangs beschriebenen Art erfindungsgemäß dadurch gelöst, daß ein Gasdurchtrittsquerschnitt des Sterilströmungspfads veränderbar ist zum Ausbilden des Überdruckströmungspfads.

Es ist somit kein Sterilströmungspfad vorgesehen, der in seinem Aufbau unveränderlich ist, sondern ein einen variablen Strömungsquerschnitt aufweisender Sterilströmungspfad. Wird eine Mindestdruckdifferenz zwischen in der Umgebung und im Aufnahmeraum des Sterilbehälters herrschenden Drücken überschritten, dann ist es bei einem erfindungsgemäßen Sterilbehälter möglich, den Gasdurchtrittsquerschnitt des Sterilströmungspfads zu verändern, insbesondere zu vergrößern, um so einen Nebenstrompfad für einen zum Abbau der herrschenden Druckdifferenz erforderlichen Gasaustausch zu öffnen. Durch Veränderung des Gasdurchtrittsquerschnitts des Sterilströmungspfads wird somit ein Überdruckströmungspfad ausgebildet. Dabei könnte der Sterilströmungspfad vom Überdruckströmungspfad getrennt sein oder auch mit diesem in Fluidverbindung stehen.

Vorteilhaft ist es, wenn der Sterilströmungspfad in der Sterilstellung einen ersten Durchtrittsquerschnitt aufweist, wenn der Sterilströmungspfad in der Überdruckstellung einen zweiten Durchtrittsquerschnitt aufweist und wenn der Überdruckströmungspfad einen dritten Durchtrittsquerschnitt aufweist, welcher der Differenz des ersten und des zweiten Durchtrittsquerschnitt entspricht. Dies bedeutet, daß sich der Überdruckströmungspfad rein rechnerisch aus einer Differenz aus zwei unterschiedlichen Durchtrittsquerschnitten des Sterilströmungspfades ergibt. Dies ist insbesondere dann der Fall, wenn der Sterilströmungspfad und der Überdruckströmungspfad miteinander in Fluidverbindung stehen oder der Überdruckströmungspfad einen Teil des sich vergrößernden Sterilströmungspfades bildet. Insbesondere in letztgenanntem Fall wird der Aufbau des Sterilbehälters deutlich vereinfacht, da keine zusätzlichen Öffnungen am Sterilbehälter vorgesehen werden müssen.

Besonders geschützt gegen äußere Einflüsse ist die Sterilbarriere, wenn sie auf einer Innenseite des Sterilbehälters angeordnet ist.

Vorzugsweise ist eine Halterung zum Halten mindestens eines Teils der Sterilbarriere am Sterilbehälter vorgesehen. Dies ermöglicht es, die Sterilbarriere auf einfache Weise am Sterilbehälter anzuordnen, beispielsweise an diesem zu lagern oder mit diesem zu verbinden.

Gemäß einer bevorzugten Ausführungsform der Erfindung kann vorgesehen sein, daß mindestens ein Teil der Sterilbarriere und die Halterung lösbar verbindbar sind, daß der mindestens eine Teil der Sterilbarriere in einer Entnahmestellung von der Halterung lösbar und in einer Verbindungsstellung an der Halterung gehalten ist. Dies gestattet es, zumindest einen Teil der Sterilbarriere zu Reinigungszwecken vom Sterilbehälter zu lösen.

Günstig ist es, wenn die Sterilbarriere in Form einer Pasteurschen Schleife ausgebildet ist und wenn der Sterilströmungspfad mäanderförmig ausgebildet ist. Für diese Art der Sterilbarriere werden keine Verbrauchsmaterialien benötigt, vielmehr läßt sich eine Pasteursche Schleife auf einfache Weise reinigen, sterilisieren und nahezu beliebig oft wiederverwenden.

Vorteilhaft ist es, wenn die Pasteursche Schleife einen ersten Träger und einen zweiten, dem ersten Träger gegenüberliegenden Träger umfaßt, wenn der erste und der zweite Träger jeweils konzentrische ringförmige Vorsprünge tragen, welche in Richtung auf den jeweils anderen Träger vorstehen, und wenn jeweils ein ringförmiger Vorsprung des einen Trägers in der Sterilstellung zwischen zwei ringförmige Vorsprünge des anderen Trägers mindestens teilweise eintaucht. Aufgrund dieser Ausgestaltung wird ein mäanderförmiger Strömungspfad ausgebildet, das heißt, es existiert keine geradlinige Verbindung durch die Sterilbarriere hindurch, so daß sich keine geradlinige Strömung längs des Sterilströmungspfades in der Sterilstellung ausbilden kann. Gas und in diesem enthaltene Teilchen, beispielsweise Keime und Bakterien, werden beim Durchströmen des Sterilströmungspfads aufeinanderfolgenden Richtungswechseln unterworfen, wobei sich schwerere Teilchen in strömungsfreien Bereichen des Strömungspfads ablagern.

Ein besonders einfacher Aufbau des Sterilströmungspfads wird erreicht, wenn die ringförmigen Vorsprünge des einen Trägers eine Wandstärke aufweisen, die kleiner ist als ein Abstand benachbarter ringförmiger Vorsprünge des anderen Trägers.

Günstigerweise weisen die ringförmigen Vorsprünge der beiden Träger eine Höhe auf, die kleiner ist als ein Abstand der beiden Träger voneinander in der Sterilstellung. Auf diese Weise wird sichergestellt, daß der Sterilströmungspfad für einen Gasaustausch dauerhaft geöffnet ist.

Eine besonders kompakte Bauform der Sterilbarriere und ein besonders einfacher Aufbau derselben lassen sich erreichen, wenn der erste Träger und der zweite Träger parallel oder im wesentlichen parallel zueinander angeordnet sind.

Ein für einen Gasaustausch ausreichender erster Gasdurchtrittsquerschnitt des Sterilströmungspfads wird sichergestellt, wenn in der Sterilstellung ein Abstand des einen Trägers von den ringförmigen Vorsprüngen des anderen Trägers kleiner ist als eine Höhe der ringförmigen Vorsprünge des einen Trägers.

Damit ein großer Luftmassenaustausch zwischen der Umgebung und dem Innenraum des Sterilbehälters gewährleistet werden kann, ist es vorteilhaft, wenn in der Überdruckstellung ein Abstand des einen Trägers von den ringförmigen Vorsprüngen des anderen Träger größer ist als eine Höhe der ringförmigen Vorsprünge des einen Trägers.

Um die Zahl der beweglichen Teile zu reduzieren, ist es vorteilhaft, wenn einer der zwei Träger unbeweglich mit dem Sterilbehälter verbunden ist.

Auf eine unter Umständen störanfällige Verbindung kann ferner verzichtet werden, wenn der eine der beiden Träger einstückig mit dem Sterilbehälter ausgebildet ist.

Zur Herstellung einer Fluidverbindung mit der Umgebung des Sterilbehälters ist es vorteilhaft, wenn einer der beiden Träger eine mit der Umgebung des Sterilbehälters in Fluidverbindung stehende Gasaustauschöffnung aufweist und wenn die ringförmigen Vorsprünge des einen der beiden Träger die Gasaustauschöffnung konzentrisch umgeben. Dies ermöglicht es insbesondere, daß nur eine einzige Öffnung am Sterilbehälter vorgesehen werden muß, welche von die Sterilbarriere ausbildenden Strukturen eines Teils der Sterilbarriere umgeben sein kann. Dies vereinfacht zusätzlich den Aufbau des Sterilbehälters und der Sterilbarriere.

Um einen Gasdurchtrittsquerschnitt des Sterilströmungspfads auf besonders einfache Weise zu vergrößern, ist es günstig, wenn der zweite Träger relativ zum ersten Träger bewegbar am Sterilbehälter gelagert ist. Eine Änderung des Gasdurchtrittsquerschnitts des Sterilströmungspfads läßt sich bei dieser Ausgestaltung dadurch erreichen, daß die beiden Träger relativ zueinander bewegt werden.

Günstigerweise ist mindestens ein Anschlag vorgesehen zum Vorgeben eines minimalen Abstandes zwischen dem ersten und dem zweiten Träger. Dadurch kann verhindert werden, daß sich die beiden Träger so weit annähern, daß ein Sterilströmungspfad vollständig geschlossen wird, was grundsätzlich nicht, in Ausnahmefällen jedoch erwünscht sein kann. Üblicherweise soll der Sterilströmungspfad jedoch dauerhaft geöffnet sein, um einen dauerhaften Gasaustausch zwischen der Umgebung des Sterilbehälters und dem Aufnahmeraum desselben zu gestatten.

Grundsätzlich wäre es denkbar, daß der erste Träger den mindestens einen Anschlag trägt. Besonders günstig ist es jedoch, wenn der zweite Träger den mindestens einen Anschlag trägt. Insbesondere dann, wenn der beweglich gelagerte Träger den mindestens einen Anschlag trägt, kann dieser sowohl zum Sicherstellen eines Abstands zwischen den beiden Träger als auch zu deren Zentrierung relativ zueinander eingesetzt werden, was insbesondere bei einer Sterilbarriere in Form einer Pasteurschen Schleife günstig ist.

Der Aufbau der Sterilbarriere wird besonders einfach, wenn der mindestens eine Anschlag in Form eines Vorsprungs ausgebildet ist und wenn eine Höhe des Vorsprungs dem minimalen Abstand zwischen dem ersten und dem zweiten Träger entspricht. Einer der beiden Träger kann somit direkt am Anschlag zur Anlage kommen.

Besonders einfach in der Herstellung wird ein Sterilbehälter, wenn die Sterilbarriere und/oder die Gasaustauschöffnung im wesentlichen kreisförmig ausgebildet sind.

Um die Sterilbarriere oder mindestens einen Teil derselben auf einfache Weise am Sterilbehälter zu halten, kann es günstig sein, wenn die Halterung mindestens ein Halteelement zum Halten und/oder Stützen mindestens eines Teils der Sterilbarriere umfaßt.

Gemäß einer bevorzugten Ausführungsform der Erfindung kann vorgesehen sein, daß das mindestens eine Halteelement am Sterilbehälter beweglich gelagert ist. Dies gestattet es insbesondere, daß mindestens ein Teil der Sterilbarriere unbeweglich mit einem Halteelement verbunden wird, so daß der mindestens eine Teil der Sterilbarriere relativ zum Sterilbehälter dann immer noch beweglich gelagert ist.

Vorteilhaft ist es, wenn das mindestens eine Halteelement unter Vorspannung am Sterilbehälter gehalten ist, so daß der Sterilbehälter bei einer Druckdifferenz, die kleiner als die Mindestdruckdifferenz ist, die Sterilstellung einnimmt. Auf diese Weise wird sichergestellt, daß der Überdruckströmungspfad nur dann geöffnet wird, wenn er tatsächlich benötigt wird, nämlich dann, wenn die Mindestdruckdifferenz überschritten wird.

Gemäß einer weiteren bevorzugten Ausführungsform der Erfindung kann vorgesehen sein, daß die Sterilbarriere mindestens einen Halteabschnitt aufweist, daß die Halterung mindestens ein Halteelement umfaßt und daß sich der mindestens eine Halteabschnitt an dem mindestens einen Halteelement abstützt. Dadurch kann die Sterilbarriere oder ein Teil derselben in definierter Weise am Sterilbehälter gehalten werden, insbesondere mit diesem verbunden oder an diesem beweglich gelagert.

Eine besonders einfache Möglichkeit der Halterung ergibt sich, wenn das mindestens eine Halteelement einen den Halteabschnitt überdeckenden Haltearm aufweist, welcher parallel oder im wesentlichen parallel zu einer die Sterilbarriere tragenden Wand des Sterilbehälters verlaufend angeordnet ist. Beispielsweise kann der Halteabschnitt vom Haltearm in der Schließstellung klemmend an einer Wand des Sterilbehälters gehalten werden.

Günstigerweise erstreckt sich das mindestens eine Halteelement über einen Winkelbereich in Umfangsrichtung der Sterilbarriere. Dadurch wird verhindert, daß sich die Sterilbarriere oder ein Teil derselben in unerwünschter Weise relativ zum Sterilbehälter, insbesondere parallel zu einer Wand desselben, bewegen kann. Das mindestens eine Halteelement dient somit auch als eine Art Zentrierung.

Um mehrere und möglichst kleine Halteelemente verwenden zu können, ist es günstig, wenn der Winkelbereich Werte von 10° bis 50° aufweist, insbesondere 20°.

Grundsätzlich wäre es möglich, ein einziges Halteelement vorzusehen. Ein besonders sicherer Halt der Sterilbarriere am Sterilbehälter wird jedoch gewährleistet, wenn mindestens zwei Halteelemente vorgesehen sind und wenn die mindestens zwei Halteelemente symmetrisch um die Sterilbarriere angeordnet sind. Insbesondere ist es vorteilhaft, vier Halteelemente symmetrisch um die Sterilbarriere herum anzuordnen.

Grundsätzlich wäre es möglich, die Sterilbarriere an einer Behälterwand oder am Behälterboden anzuordnen. Besonders leicht zugänglich ist sie jedoch, insbesondere zu Zwecken der Reinigung, wenn sie am Deckel angeordnet ist.

Damit bei einem schlagartigen Öffnen des Überdruckströmungspfads die Sterilbarriere und die im Aufnahmeraum enthaltenen Gegenstände nicht zerstört werden kann, beispielsweise durch unbeabsichtigtes Lösen eines Teils der Sterilbarriere von einer diese haltenden Halterung, kann es günstig sein, wenn mindestens ein Anschlag vorgesehen ist zum Begrenzen eines maximalen Gasdurchtrittsquerschnitts des Sterilströmungspfads.

Der Sterilbehälter wird besonders leicht und ist einfach herzustellen, wenn der Deckel und/oder die Sterilbarriere aus einem Kunststoff hergestellt ist, insbesondere aus Polyetheretherketon (PEEK) oder Polyphenylensulfon (PPSU). Denkbar wäre es auch, den Kunststoff zusätzlich zu verstärken, beispielsweise durch Glasfasern und/oder Kohlefasern.

Vorteilhaft ist es, wenn in der Überdruckstellung der in der Umgebung des Sterilbehälters herrschende Druck den im Aufnahmeraum herrschenden Druck mindestens um die Mindestdruckdifferenz übersteigt. Dies bedeutet, daß der Überdruckströmungspfad mindestens teilweise geöffnet ist, wenn in der Umgebung des Sterilbehälters ein Druck herrscht, welcher mindestens um die Mindestdruckdifferenz größer ist als der im Aufnahmeraum herrschende Druck. Dadurch kann eine beispielsweise beim Sterilisieren des Sterilbehälters herrschende Druckdifferenz abgebaut werden, indem der Überdruckströmungspfad mindestens teilweise geöffnet wird und so das Einströmen von Heißdampf in den Aufnahmeraum hinein gestattet. Der variable Strömungsquerschnitt ermöglicht daher die Ausbildung einer Art Überdruckventil in Form eines Einlaßventils.

Die nachfolgende Beschreibung einer bevorzugten Ausführungsform der Erfindung dient im Zusammenhang mit der Zeichnung der näheren Erläuterung. Es zeigen:
- Figur 1:: eine teilweise Schnittansicht durch einen Sterilbehälter;
- Figur 2:: eine vergrößerte Ansicht des Bereichs A in Figur 1 mit einer Ste- rilbarriere in der Sterilstellung;
- Figur 3:: eine Ansicht ähnlich Figur 2 mit der Sterilbarriere in der Über- druckstellung; und

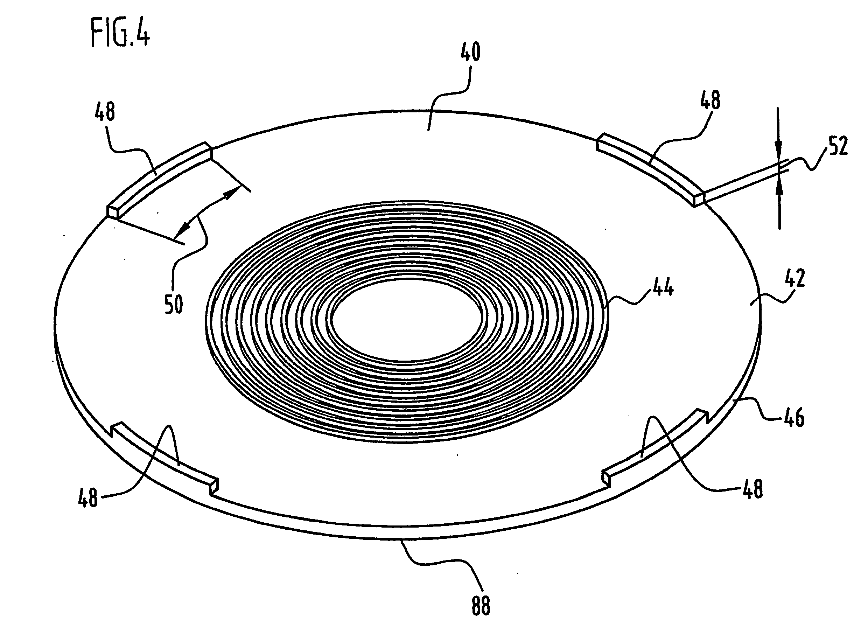

- Figur 4:: eine perspektivische Ansicht eines beweglich gelagerten Teils der Sterilbarriere.

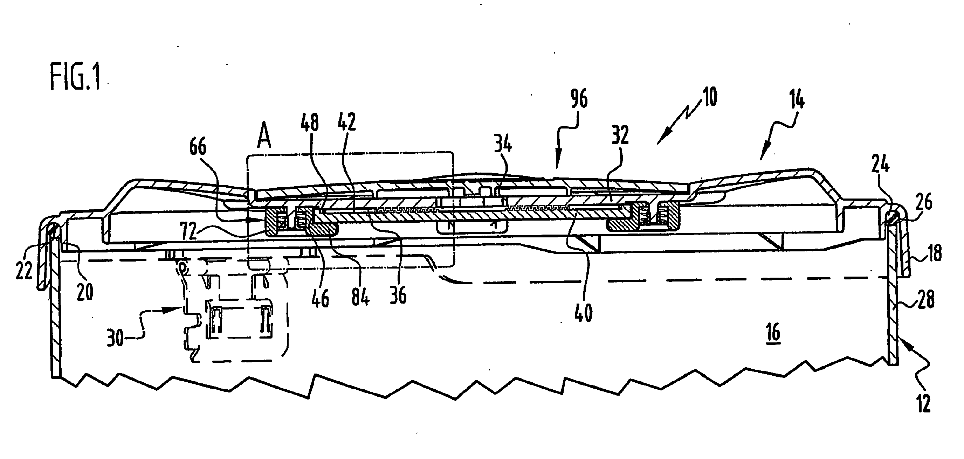

In Figur 1 ist ein insgesamt mit dem Bezugszeichen 10 versehener Sterilcontainer dargestellt, welcher eine Behälterwanne 12 und einen Deckel 14 zum Verschließen derselben umfaßt. In einem Innenraum 16 des Sterilcontainers 10 können beispielsweise chirurgische Instrumente und chirurgisches Material gelagert werden.

Der Deckel 14 ist mit einem senkrecht abstehenden, umlaufenden Rand 18 versehen und mit einem parallel zu diesem verlaufenden, etwas kürzeren Vorsprung 20. Der Rand 18 und der Vorsprung 20 definieren zwischen sich eine umlaufende Dichtnut 22, in welche eine Dichtung 24 eingesetzt ist. Die Dichtnut 22 dient zur Aufnahme von Stirnkanten 26 von Wänden 28 der Behälterwanne 12. Die Dichtung 24 kommt direkt auf den Stirnkanten 26 zu liegen und wird mittels zweier, einander gegenüberliegend, am Deckel 14 angeordneter Verschlußklappen 30 beim Verriegeln der Behälterwanne 12 etwas komprimiert, so daß der Deckel 14 die Behälterwanne 12 gasdicht verschließt.

Zentral mittig ist am Deckel 14 in einer Deckelwand 32 eine kreisförmige Einlaßöffnung 34 vorgesehen, welche von auf einer Innenfläche 36 der Deckelwand 32 senkrecht abstehenden konzentrischen Ringvorsprüngen 38 umgeben ist. Die Deckelwand 32 bildet somit einen ersten Träger für die Ringvorsprünge 38. Einen zweiten Träger bildet eine Trägerplatte 40 in Form einer flachen Scheibe. Von einer der Innenfläche 36 zugewandten Seitenfläche 42 der Trägerplatte 40 stehen konzentrische, ringförmige Vorsprünge 44 ab, deren Wandstärke kleiner ist als ein Abstand jeweils benachbarter Vorsprünge 44. Ebenso ist eine Wandstärke der Ringvorsprünge 38 kleiner als ein Abstand zwischen zwei benachbarten Ringvorsprüngen 38.

Die Radien der Ringvorsprünge 38 und der Vorsprünge 44 sind so gewählt, daß sowohl die Ringvorsprünge 38 als auch die Vorsprünge 44 konzentrisch ausgerichtet sind, wobei jeweils ein Ringvorsprung 38 teilweise zwischen zwei Vorsprünge 44 eintaucht beziehungsweise ein Vorsprung 44 zwischen zwei Ringvorsprünge 38.

Ferner sind auf der Seitenfläche 42 benachbart einer Seitenkante 46 der Trägerplatte 40 vier Abstandshalter 48 von der Seitenfläche 42 abstehend angeordnet, die gleichmäßig über den Umfang der Trägerplatte 40 verteilt sind und sich jeweils über einen Winkelbereich 50 von etwa 20° erstrecken. Eine Höhe der Abstandshalter 48 ausgehend von der Seitenfläche 42 ist sowohl größer als eine Höhe 52 der Ringvorsprünge 38 ausgehend von der Innenfläche 36 als auch eine Höhe 56 der Vorsprünge 44 ausgehend von der Seitenfläche 42.

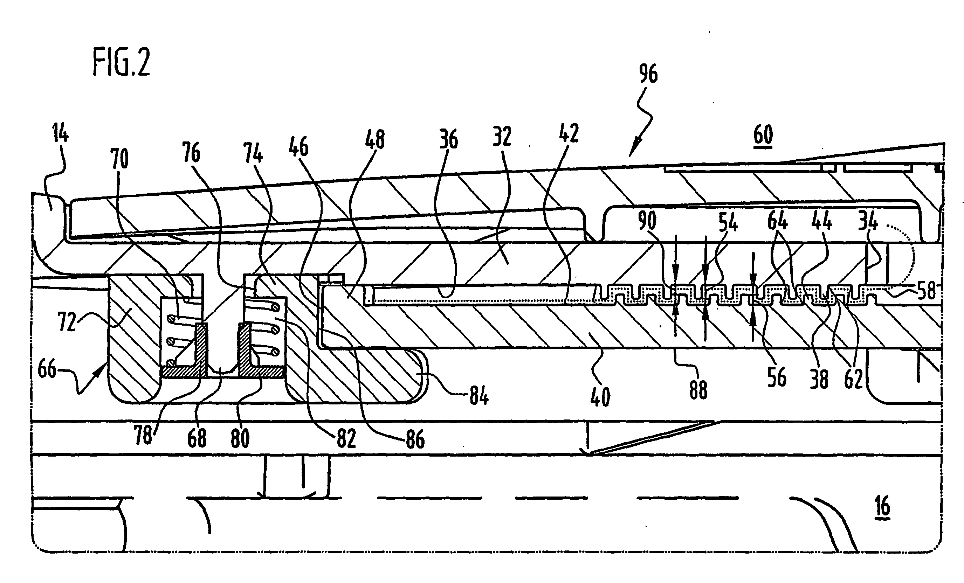

Die Abstandshalter 48, welche an der.Innenfläche 36 anliegen, definieren einen minimalen Abstand der Innenfläche 36 von der Seitenfläche 42. In dieser in den Figuren 1 und 2 dargestellten Sterilstellung wird somit ein mäanderförmiger Strömungspfad 58 ausgebildet, der in Figur 2 gepunktet dargestellt ist. Er verbindet den Innenraum 16 des Sterilcontainers 10 mit einer Umgebung 60 desselben. Der mäanderförmige Strömungspfad 58 wird auch als Pasteursche Schleife bezeichnet und weist einen besonderen Effekt auf, nämlich den, daß in einem Gasstrom, welcher entlang des Strömungspfads 58 strömt, mitgeführte Teilchen, beispielsweise Keime und Bakterien, sich in Ecken 62 im Übergangsbereich zwischen den Ringvorsprüngen 38 und der Innenfläche 36 sowie in Ecken 64 im Übergangsbereich zwischen den Vorsprüngen 44 und der Seitenfläche 42 ablagern, da in den genannten Bereichen keine Strömung existiert. Durch die Vielzahl der Windungen des Strömungspfads 58 und folglich die Vielzahl der strömungslosen Bereiche werden im Gasstrom mitgeführte Teilchen quasi durch Ablagerung in den Ecken 62 und 64 ausgefiltert.

Die Trägerplatte 40 ist mittels vier identischer Halterungen 66 beweglich an der Deckelwand 32 gelagert. Jede der Halterungen 66 weist einen senkrecht von der Innenfläche 36 abstehenden Lagerzapfen 68 auf. Dieser ist von einem topfförmigen Lagerelement 72 umgeben, welches einen an der Innenfläche 36 anliegenden Boden 74 aufweist. Der Boden 74 wiederum ist mit einer Bohrung 76 versehen, die im Durchmesser etwas größer ist als ein Durchmesser des Lagerzapfens 68. Auf den Lagerzapfen 68 ist eine Abschlußhülse mit einem radial abstehenden Ringflansch 80 aufgesetzt, dessen Durchmesser in etwa einem Innendurchmesser des Lagerelements 72 entspricht. Auf diese Weise wird vom Ringflansch 80 der Abschlußhülse 78 und dem Lagerelement 72 ein den Lagerzapfen 68 umgebender Ringraum 82 begrenzt, in welchen eine Schraubenfeder 70 eingesetzt ist, die sich einerseits am Ringflansch 80, andererseits am Boden 74 des Lagerelements 72 abstützt. Durch diese besondere Anordnung wird das Lagerelement durch die Schraubenfeder 70 in einer Grundstellung, die der Sterilstellung entspricht, unter Vorspannung gegen die Innenfläche 36 gedrückt.

Vom Lagerelement 72 steht quer, das heißt parallel zur Innenfläche 36, ein Lagervorsprung 84 ab, so daß das Lagerelement 72 zusammen mit der Innenfläche 36 eine nutförmige Aufnahme 86 zur Lagerung der Trägerplatte 40 bildet. Der Lagervorsprung 84 liegt dabei an einer Unterseite 88 der Trägerplatte 40 an. Durch die Wirkung der Schraubenfeder 70 wird der Lagervorsprung 84 gegen die Unterseite 88 gedrückt, so daß die Trägerplatte 40 mit den Abstandhaltern 48 in der Grundstellung an der Innenfläche 36 anschlägt.

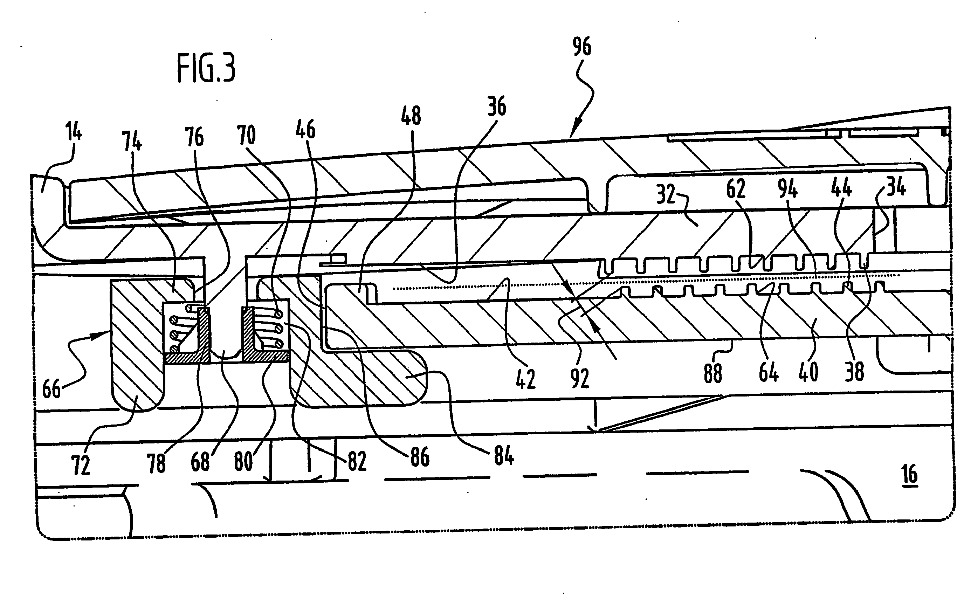

In der in Figur 2 dargestellten Grund- oder Sterilstellung weist der Strömungspfad 58 einen mit 90 bezeichneten Querschnitt auf. Erhöht sich der Druck in der Umgebung 60 des Sterilcontainers 10 gegenüber einem Druck im Innenraum 16, so wird die Trägerplatte 40 gegen die Lagervorsprünge 84 gedrückt, wodurch der Boden des Lagerelements 72 die sich am Ringflansch 80 abstützende Schraubenfeder 70 zusammendrückt. Dadurch vergrößert sich ein Abstand zwischen der Seitenfläche 42 und der Innenfläche 36, so daß der Strömungspfad 58 einen Querschnitt 92 aufweist, welcher größer ist als der Querschnitt 90. Durch die Vergrößerung des Querschnitts 90 des Strömungspfads .. 58 wird ein geradliniger Strömungspfad 94 zwischen den Ringvorsprüngen 38 und den Vorsprüngen 44 ausgebildet, mittels dem ein Abbau der. übermäßigen Druckdifferenz erfolgen kann. Der Strömungspfad 94 bildet somit einen Überdruckströmungspfad. Die Wirkung der Pasteurschen Schleife aufgrund des mäanderförmigen Strömungspfads 58 wird in dieser, in Figur 3 dargestellten Überdruckstellung abgeschaltet. Geht der auf die Trägerplatte 40 wirkende Druck wieder zurück, drücken die Schraubenfedern 70 die Lagerelemente 72 wieder gegen die Innenfläche 36, so daß die von der Trägerplatte 40 zusammen mit den Ringvorsprüngen 38 und den Vorsprüngen 44 gebildete Sterilbarriere wieder in die in Figur 2 dargestellte Sterilstellung überführt wird.

Alle Elemente des Deckels sind vorzugsweise aus einem Kunststoff gefertigt, so daß eine Korrosion des Deckels 14 minimiert wird.

Auf einer Außenseite des Deckels 14 ist die Einlaßöffnung 34 vollständig überdeckend ein Schutzdeckel 96 in nicht näher dargestellter Weise an den Deckel 14 angeclipst.

## Patentansprüche

1. Sterilbehälter (10), insbesondere zur Aufnahme und sterilen Aufbewahrung von chirurgischem Besteck oder chirurgischem Material, mit einem durch einen Behälterboden und Behälterwände (28) gebildeten Aufnahmeraum (16), mit einem Deckel (14) zum Verschließen des Aufnahmeraums (16), mit einer Sterilbarriere (36, 38, 40, 44), welche dauerhaft einen Sterilströmungspfad (58) zum Herstellen einer Fluidverbindung zwischen dem Aufnahmeraum (16) und einer Umgebung (60) des Sterilbehälters (10) definiert, und mit einem Überdruckströmungspfad (94), welcher eine Fluidverbindung zwischen dem Aufnahmeraum (16) und der Umgebung (60) des Sterilbehälters (10) definiert, wobei in einer Sterilstellung des Sterilbehälters (10), in welcher ein Gasaustausch zwischen dem Aufnahmeraum (16) und der Umgebung (60) des Sterilbehälters (10) nur durch den Sterilströmungspfad (58) möglich ist, der Überdruckströmungspfad (94) geschlossen ist und wobei in einer Überdruckstellung des Sterilbehälters (10), in welcher eine Druckdifferenz zwischen im Aufnahmeraum (16) und in der Umgebung (60) des Sterilbehälters (10) herrschenden Drücken eine Mindestdruckdifferenz übersteigt, der Überdruckströmungspfad (94) mindestens teilweise geöffnet ist, **dadurch gekennzeichnet, daß** ein Gasdurchtrittsquerschnitt (90, 92) des Sterilströmungspfads (58) veränderbar ist zum Ausbilden des Überdruckströmungspfads (94).

2. Sterilbehälter nach Anspruch 1, **dadurch gekennzeichnet, daß** der Sterilströmungspfad (58) in der Sterilstellung einen ersten Durchtrittsquerschnitt (90) aufweist, daß der Sterilströmungspfad (58) in der Überdruckstellung einen zweiten Durchtrittsquerschnitt (92) aufweist und daß der Überdruckströmungspfad (94) einen dritten Durchtrittsquerschnitt aufweist, welcher der Differenz des ersten und des zweiten Durchtrittsquerschnitts (90, 92) entspricht.

3. Sterilbehälter nach einem der voranstehenden Ansprüche, **dadurch gekennzeichnet, daß** die Sterilbarriere (36, 38, 40, 44) auf einer Innenseite des Sterilbehälters (10) angeordnet ist.

4. Sterilbehälter nach einem der voranstehenden Ansprüche, **dadurch gekennzeichnet, daß** eine Halterung (66) zum Halten mindestens eines Teils (40, 44) der Sterilbarriere (36, 38, 40, 44) am Sterilbehälter vorgesehen ist.

5. Sterilbehälter nach Anspruch 4, **dadurch gekennzeichnet, daß** mindestens ein Teil der Sterilbarriere (40, 44) und die Halterung (66) lösbar verbindbar sind, daß der mindestens eine Teil (40, 44) der Sterilbarriere (36, 38, 40, 44) in einer Entnahmestellung von der Halterung (66) lösbar und in einer Verbindungsstellung an der Halterung (66) gehalten ist.

6. Sterilbehälter nach einem der voranstehenden Ansprüche, **dadurch gekennzeichnet, daß** die Sterilbarriere (36, 38, 40, 44) in Form einer Pasteurschen Schleife ausgebildet ist und daß der Sterilströmungspfad (58) mäanderförmig ausgebildet ist.

7. Sterilbehälter nach Anspruch 6, **dadurch gekennzeichnet, daß** die Pasteursche Schleife (36, 38, 40, 44) einen ersten Träger (36) und einen zweiten, dem ersten Träger (36) gegenüberliegenden Träger (40) umfaßt, daß der erste und der zweite Träger (36, 40) jeweils konzentrische ringförmige Vorsprünge (38, 44) tragen, welche in Richtung auf den jeweils anderen Träger (36, 40) vorstehen, und daß jeweils ein ringförmiger Vorsprung (38, 44) des einen Trägers (36, 40) in der Sterilstellung zwischen zwei ringförmige Vorsprünge (44, 38) des anderen Trägers (40, 36) mindestens teilweise eintaucht.

8. Sterilbehälter nach Anspruch :7, **dadurch gekennzeichnet, daß** die ringförmigen Vorsprünge (38, 44) des einen Trägers (36, 40) eine Wandstärke aufweisen, die kleiner ist als ein Abstand benachbarter ringförmiger Vorsprünge (44, 38) des anderen Trägers (40, 36).

9. Sterilbehälter nach einem der Ansprüche 7 oder 8, **dadurch gekennzeichnet, daß** die ringförmigen Vorsprünge (38, 44) der beiden Träger (36, 40) eine Höhe (54, 56) aufweisen, die kleiner ist als ein Abstand (52) der beiden Träger (36, 40) voneinander in der Sterilstellung.

10. Sterilbehälter nach einem der Ansprüche 7 bis 9, **dadurch gekennzeichnet, daß** der erste Träger (36) und der zweite Träger (40) parallel oder im wesentlichen parallel zueinander angeordnet sind.

11. Sterilbehälter nach einem der Ansprüche 7 bis 10, **dadurch gekennzeichnet, daß** in der Sterilstellung ein Abstand des einen Trägers (36, 40) von den ringförmigen Vorsprüngen (44, 38) des anderen Trägers (40, 36) kleiner ist als eine Höhe der ringförmigen Vorsprünge (38, 44) des einen Trägers (36, 40).

12. Sterilbehälter nach einem der Ansprüche 7 bis 11, **dadurch gekennzeichnet, daß** in der Überdruckstellung ein Abstand des einen Trägers (36, 40) von den ringförmigen Vorsprüngen (44, 38) des anderen Trägers (40, 36) größer ist als eine Höhe der ringförmigen Vorsprünge (38, 44) des einen Trägers (36, 40).

13. Sterilbehälter nach einem der Ansprüche 7 bis 12, **dadurch gekennzeichnet, daß** einer (36) der zwei Träger (36, 40) unbeweglich mit dem Sterilbehälter (10) verbunden ist.

14. Sterilbehälter nach Anspruch 13, **dadurch gekennzeichnet, daß** der eine (36) der zwei Träger einstückig mit dem Sterilbehälter (10) ausgebildet ist.

15. Sterilbehälter nach einem der Ansprüche 7 bis 14, **dadurch gekennzeichnet, daß** einer (36) der beiden Träger (36, 40) eine mit der Umgebung (60) des Sterilbehälters (10) in Fluidverbindung stehende Gasaustauschöffnung (34) aufweist und daß die ringförmigen Vorsprünge (38) des einen (36) der beiden Träger (36, 40) die Gasaustauschöffnung (34) konzentrisch umgeben.

16. Sterilbehälter nach einem der Ansprüche 7 bis 15, **dadurch gekennzeichnet, daß** der zweite Träger (40) relativ zum ersten Träger (36) bewegbar am Sterilbehälter (10) gelagert ist.

17. Sterilbehälter nach einem der Ansprüche 7 bis 16, **dadurch gekennzeichnet, daß** mindestens ein Anschlag (48) vorgesehen ist zum Vorgeben eines minimalen Abstandes (52) zwischen dem ersten und dem zweiten Träger (36, 40).

18. Sterilbehälter nach Anspruch 17, **dadurch gekennzeichnet, daß** der zweite Träger (40) den mindestens einen Anschlag (48) trägt.

19. Sterilbehälter nach Anspruch 17 oder 18, **dadurch gekennzeichnet, daß** der mindestens eine Anschlag (48) in Form eines.Vorsprungs (48) ausgebildet ist und daß eine Höhe (52) des Vorsprungs (48) dem minimalen Abstand (52) zwischen dem ersten und dem zweiten Träger (36, 40) entspricht.

20. Sterilbehälter nach einem der voranstehenden Ansprüche, **dadurch gekennzeichnet, daß** die Sterilbarriere (36, 38, 40, 44) und/oder die Gasaustauschöffnung (34) kreisförmig oder im wesentlichen kreisförmig ausgebildet sind.

21. Sterilbehälter nach einem der Ansprüche 4 bis 20, **dadurch gekennzeichnet, daß** die Halterung (66) mindestens ein Halteelement (72) zum Halten und/oder Stützen mindestens eines Teils (40, 44) der Sterilbarriere (36, 38, 40, 44) umfaßt.

22. Sterilbehälter nach Anspruch 21, **dadurch gekennzeichnet, daß** das mindestens eine Halteelement (72) am Sterilbehälter (10) beweglich gelagert ist.

23. Sterilbehälter nach einem der Ansprüche 21 oder 22, **dadurch gekennzeichnet, daß** das mindestens eine Halteelement (72) unter Vorspannung am Sterilbehälter (10) gehalten ist, so daß der Sterilbehälter (10) bei einer Druckdifferenz, die kleiner als die Mindestdruckdifferenz ist, die Sterilstellung einnimmt.

24. Sterilbehälter nach einem der Ansprüche 21 bis 23, **dadurch gekennzeichnet, daß** die Sterilbarriere (36, 38, 40, 44) mindestens einen Halteabschnitt aufweist, daß die Halterung (66) mindestens ein Halteelement umfaßt und daß sich der mindestens eine Halteabschnitt an dem mindestens einen Halteelement (84) abstützt.

25. Sterilbehälter nach Anspruch 24, **dadurch gekennzeichnet, daß** das mindestens eine Halteelement (72) einen den Halteabschnitt überdeckenden Haltearm (84) aufweist, welcher parallel oder im wesentlichen parallel zu einer die Sterilbarriere (36, 38, 40, 44) tragenden Wand (32) des Sterilbehälters (10) verlaufend angeordnet ist.

26. Sterilbehälter nach einem der Ansprüche 21 bis 25, **dadurch gekennzeichnet, daß** sich das mindestens eine Halteelement (72) über einen Winkelbereich in Umfangsrichtung der Sterilbarriere (36, 38, 40, 44) erstreckt.

27. Sterilbehälter nach Anspruch 26, **dadurch gekennzeichnet, daß** der Winkelbereich Werte von 10° bis 50° aufweist, insbesondere 20°.

28. Sterilbehälter nach einem der Ansprüche 21 bis 27, **dadurch gekennzeichnet, daß** mindestens zwei Halteelemente (72) vorgesehen sind und daß die mindestens zwei Halteelemente (72) symmetrisch um die Sterilbarriere (36, 38, 40, 44) herum angeordnet sind.

29. Sterilbehälter nach einem der voranstehenden Ansprüche, **dadurch gekennzeichnet, daß** die Sterilbarriere (36, 38, 40, 44) am Deckel (14) angeordnet ist.

30. Sterilbehälter nach einem der voranstehenden Ansprüche, **dadurch gekennzeichnet, daß** mindestens ein Anschlag (80) vorgesehen ist zum Begrenzen eines maximalen Gasdurchtrittsquerschnitts (92) des Sterilströmungspfads (58).

31. Sterilbehälter nach einem der voranstehenden Ansprüche, **dadurch gekennzeichnet, daß** der Deckel (14) und/oder die Sterilbarriere (36, 38, 40, 44) aus einem Kunststoff hergestellt ist, insbesondere aus Polyetheretherketon (PEEK) oder Polyphenylensulfon (PPSU).

32. Sterilbehälter nach einem der voranstehenden Ansprüche, **dadurch gekennzeichnet, daß** in der Überdruckstellung der in der Umgebung des Sterilbehälters (10) herrschende Druck den im Aufnahmeraum (16) herrschenden Druck mindestens um die Mindestdruckdifferenz übersteigt.

## Claims

1. Sterile container (10), in particular, for receiving and storing surgical instruments or surgical material under sterile conditions, comprising a receiving space (16) formed by a container bottom and container walls (28), a lid (14) for closing the receiving space (16), a sterile barrier (36, 38, 40, 44) permanently defining a sterile flow path (58) for establishing a fluid connection between the receiving space (16) and an environment (60) outside of the sterile container (10), and an overpressure flow path (94) defining a fluid connection between the receiving space (16) and the environment (60) outside of the sterile container (10), wherein the overpressure flow path (94) is closed when the sterile container (10) is in a sterile position in which an exchange of gas between the receiving space (16) and the environment (60) outside of the sterile container (10) is only possible through the sterile flow path (58), and wherein the overpressure flow path (94) is at least partially open when the sterile container (10) is in an overpressure position in which a pressure difference between pressures prevailing in the receiving space (16) and in the environment (60) outside of the sterile container (10) exceeds a minimum pressure difference, **characterized in that** a gas flow cross section (90, 92) of the sterile flow path (58) is alterable for formation of the overpressure flow path (94).

2. Sterile container in accordance with claim 1, **characterized in that** in the sterile position the sterile flow path (58) has a first flow cross section (90), **in that** in the overpressure position the sterile flow path (58) has a second flow cross section (92), and **in that** the overpressure flow path (94) has a third flow cross section, which corresponds to the difference between the first and the second flow cross sections (90, 92).

3. Sterile container in accordance with any one of the preceding claims, **characterized in that** the sterile barrier (36, 38, 40, 44) is arranged on an inner side of the sterile container (10).

4. Sterile container in accordance with any one of the preceding claims, **characterized in that** a holding device (66) is provided for holding at least one part (40, 44) of the sterile barrier (36, 38, 40, 44) on the sterile container.

5. Sterile container in accordance with claim 4, **characterized in that** at least one part of the sterile barrier (40, 44) and the holding device (66) are releasably connectable, and **in that** the at least one part (40, 44) of the sterile barrier (36, 38, 40, 44) is releasable from the holding device (66) in a remove position and is held on the holding device (66) in a connect position.

6. Sterile container in accordance with any one of the preceding claims, **characterized in that** the sterile barrier (36, 38, 40, 44) is constructed in the form of a Pasteurian loop (tortuous path), and **in that** the sterile flow path (58) is of meander-shaped configuration.

7. Sterile container in accordance with claim 6, **characterized in that** the Pasteurian loop (tortuous path) (36, 38, 40, 44) comprises a first carrier (36) and a second carrier (40) facing the first carrier (36), **in that** the first and second carriers (36, 40) each carry concentric ring-shaped projections (38, 44) which extend in the direction towards the respective other carrier (36, 40), and **in that** in the sterile position a ring-shaped projection (38, 44) of the one carrier (36, 40) respectively enters at least partially in between two ring-shaped projections (44, 38) of the other carrier (40, 36).

8. Sterile container in accordance with claim 7, **characterized in that** the ring-shaped projections (38, 44) of the one carrier (36, 40) have a wall thickness which is smaller than a spacing between adjacent ring-shaped projections (44, 38) of the other carrier (40, 36).

9. Sterile container in accordance with claim 7 or 8, **characterized in that** the ring-shaped projections (38, 44) of the two carriers (36, 40) have a height (54, 56) which is smaller than a spacing (52) of the two carriers (36, 40) from one another in the sterile position.

10. Sterile container in accordance with any one of claims 7 to 9, **characterized in that** the first carrier (36) and the second carrier (40) are arranged parallel or substantially parallel to one another.

11. Sterile container in accordance with any one of claims 7 to 10, **characterized in that** in the sterile position a spacing of the one carrier (36, 40) from the ring-shaped projections (44, 38) of the other carrier (40, 36) is smaller than a height of the ring-shaped projections (38, 44) of the one carrier (36, 40).

12. Sterile container in accordance with any one of claims 7 to 11, **characterized in that** in the overpressure position a spacing of the one carrier (36, 40) from the ring-shaped projections (44, 38) of the other carrier (40, 36) is greater than a height of the ring-shaped projections (38, 44) of the one carrier (36, 40).

13. Sterile container in accordance with any one of claims 7 to 12, **characterized in that** one (36) of the two carriers (36, 40) is immovably connected to the sterile container (10).

14. Sterile container in accordance with claim 13, **characterized in that** one (36) of the two carriers is formed integrally with the sterile container (10).

15. Sterile container in accordance with any one of claims 7 to 14, **characterized in that** one (36) of the two carriers (36, 40) has a gas exchange opening (34) which is in fluid communication with the environment (60) outside of the sterile container (10), and **in that** the ring-shaped projections (38) of one (36) of the two carriers (36, 40) concentrically surround the gas exchange opening (34).

16. Sterile container in accordance with any one of claims 7 to 15, **characterized in that** the second carrier (40) is mounted on the sterile container (10) so as to be movable relative to the first carrier (36).

17. Sterile container in accordance with any one of claims 7 to 16, **characterized in that** at least one stop (48) is provided for specifying a minimum spacing (52) between the first and the second carriers (36, 40).

18. Sterile container in accordance with claim 17, **characterized in that** the second carrier (40) carries the at least one stop (48).

19. Sterile container in accordance with claim 17 or 18, **characterized in that** the at least one stop (48) is constructed in the form of a projection (48), and **in that** a height (52) of the projection (48) corresponds to the minimum spacing (52) between the first and the second carriers (36, 40).

20. Sterile container in accordance with any one of the preceding claims, **characterized in that** the sterile barrier (36, 38, 40, 44) and/or the gas exchange opening (34) is/are of circular or substantially circular construction.

21. Sterile container in accordance with any one of claims 4 to 20, **characterized in that** the holding device (66) comprises at least one holding element (72) for holding and/or supporting at least one part (40, 44) of the sterile barrier (36, 38, 40, 44).

22. Sterile container in accordance with claim 21, **characterized in that** the at least one holding element (72) is mounted so as to be movable on the sterile container (10).

23. Sterile container in accordance with claim 21 or 22, **characterized in that** the at least one holding element (72) is held in a biased manner on the sterile container (10), so that the sterile container (10) will assume the sterile position when a pressure difference is smaller than the minimum pressure difference.

24. Sterile container in accordance with any one of claims 21 to 23, **characterized in that** the sterile barrier (36, 38, 40, 44) comprises at least one holding portion, **in that** the holding device (66) comprises at least one holding element, and **in that** the at least one holding portion is supported on the at least one holding element (84).

25. Sterile container in accordance with claim 24, **characterized in that** the at least one holding element (72) comprises a holding arm (84) which covers the holding portion and is arranged so as to extend parallel or substantially parallel to a wall (32) of the sterile container (10), which carries the sterile barrier (36, 38, 40, 44).

26. Sterile container in accordance with any one of claims 21 to 25, **characterized in that** the at least one holding element (72) extends over an angular range in the circumferential direction of the sterile barrier (36, 38, 40, 44).

27. Sterile container in accordance with claim 26, **characterized in that** the values of the angular range are from 10° to 50°, in particular, 20°.

28. Sterile container in accordance with any one of claims 21 to 27, **characterized in that** at least two holding elements (72) are provided, and **in that** the at least two holding elements (72) are arranged symmetrically around the sterile barrier (36, 38, 40, 44).

29. Sterile container in accordance with any one of the preceding claims, **characterized in that** the sterile barrier (36, 38, 40, 44) is arranged on the lid (14).

30. Sterile container in accordance with any one of the preceding claims, **characterized in that** at least one stop (80) is provided for delimiting a maximum gas flow cross section (92) of the sterile flow path (58).

31. Sterile container in accordance with any one of the preceding claims, **characterized in that** the lid (14) and/or the sterile barrier (36, 38, 40, 44) is/are made from a plastic material, in particular, from polyetheretherketone (PEEK) or polyphenylene sulfone (PPSU).

32. Sterile container in accordance with any one of the preceding claims, **characterized in that** in the overpressure position the pressure prevailing in the environment outside of the sterile container (10) exceeds the pressure prevailing in the receiving space (16) by at least the minimum pressure difference.

## Revendications

1. Récipient stérile (10), notamment destiné à recevoir et à conserver de manière stérile des instruments chirurgicaux ou du matériel chirurgical, comprenant un compartiment de réception (16) formé par un fond de récipient et des parois de récipient (28), comprenant également un couvercle (14) pour fermer le compartiment de réception (16), ainsi qu'une barrière stérile (36, 38, 40, 44), qui définit en permanence un parcours d'écoulement stérile (58) pour l'établissement d'une liaison fluidique entre le compartiment de réception (16) et un environnement (60) du récipient stérile (10), et comprenant finalement un parcours d'écoulement en pression (94), qui définit une liaison fluidique entre le compartiment de réception (16) et l'environnement (60) du récipient stérile (10), le parcours d'écoulement en pression (94) étant fermé pour une position stérile du récipient stérile (10), dans laquelle un échange de gaz entre le compartiment de réception (16) et l'environnement (60) du récipient stérile (10) n'est possible que par l'intermédiaire du parcours d'écoulement stérile (58), et le parcours d'écoulement en pression (94) étant ouvert au moins partiellement, pour une position en pression du récipient stérile (10), dans laquelle une différence de pression entre des pressions régnant dans le compartiment de réception (16) et dans l'environnement (60) du récipient stérile (10) dépasse une différence de pression minimale, **caractérisé en ce qu'**une section de passage de gaz (90, 92) du parcours d'écoulement stérile (58) peut être modifiée pour former le parcours d'écoulement en pression (94).

2. Récipient stérile selon la revendication 1, **caractérisé en ce que** le parcours d'écoulement stérile (58) présente une première section de passage (90) dans la position stérile, **en ce que** le parcours d'écoulement stérile (58) présente une deuxième section de passage (92) dans la position en pression, et **en ce que** le parcours d'écoulement en pression (94) présente une troisième section de passage, qui correspond à la différence entre la première et de la deuxième section de passage (90, 92).

3. Récipient stérile selon l'une des revendications précédentes, **caractérisé en ce que** la barrière stérile (36, 38, 40, 44) est agencée sur le côté intérieur du récipient stérile (10).

4. Récipient stérile selon l'une des revendications précédentes, **caractérisé en ce qu'**un support de maintien (66) pour maintenir au moins une partie (40, 44) de la barrière stérile (36, 38, 40, 44) est prévu sur le récipient stérile.

5. Récipient stérile selon la revendication 4, **caractérisé en ce qu'**au moins une partie de la barrière stérile (40, 44) et le support de maintien (66) peuvent être reliés de manière amovible, et **en ce que** ladite au moins une partie (40, 44) de la barrière stérile (36, 38, 40, 44) est amovible du support de maintien (66) dans une position de prélèvement, et est maintenue sur le support de maintien (66) dans une position de liaison.

6. Récipient stérile selon l'une des revendications précédentes, **caractérisé en ce que** la barrière stérile (36, 38, 40, 44) est réalisée sous la forme d'un labyrinthe de rétention de germes, et **en ce que** le parcours d'écoulement stérile (58) est d'une configuration en forme de méandres.

7. Récipient stérile selon la revendication 6, **caractérisé en ce que** le labyrinthe de rétention de germes (36, 38, 40, 44) comprend un premier support (36) et un deuxième support (40) en regard du premier support (36), **en ce que** le premier et le deuxième support (36, 40) portent chacun des protubérances (38, 44) concentriques, de forme annulaire, qui font saillie en direction respectivement de l'autre support (36, 40), et **en ce que** dans la position stérile, une protubérance annulaire (38, 44) respective d'un support (36, 40) s'engage au moins partiellement entre deux protubérances annulaires (44, 38) de l'autre support (40, 36).

8. Récipient stérile selon la revendication 7, **caractérisé en ce que** les protubérances annulaires (38, 44) d'un support (36, 40) présentent une épaisseur de paroi, qui est inférieure à la distance entre des protubérances annulaires (44, 38) voisines de l'autre support (40, 36).

9. Récipient stérile selon l'une des revendications 7 ou 8, **caractérisé en ce que** les protubérances annulaires (38, 44) des deux supports (36, 40) présentent une hauteur (54, 56) qui est inférieure à la distance (52) des deux supports (36, 40) l'un de l'autre, dans la position stérile.

10. Récipient stérile selon l'une des revendications 7 à 9, **caractérisé en ce que** le premier support (36) et le deuxième support (40) sont agencés parallèlement ou sensiblement parallèlement l'un à l'autre.

11. Récipient stérile selon l'une des revendications 7 à 10, **caractérisé en ce que** dans la position stérile, une distance d'un support (36, 40) aux protubérances annulaires (44, 38) de l'autre support (40, 36) est inférieure à une hauteur des protubérances annulaires (38, 44) dudit un support (36, 40).

12. Récipient stérile selon l'une des revendications 7 à 11, **caractérisé en ce que** dans la position en pression, une distance d'un support (36, 40) aux protubérances annulaires (44, 38) de l'autre support (40, 36) est supérieure à une hauteur des protubérances annulaires (38, 44) dudit un support (36, 40).

13. Récipient stérile selon l'une des revendications 7 à 12, **caractérisé en ce que** l'un (36) des deux supports (36, 40) est relié de manière non mobile au récipient stérile (10).

14. Récipient stérile selon la revendication 13, **caractérisé en ce que** l'un (36) des deux supports est réalisé d'un seul tenant avec le récipient stérile (10).

15. Récipient stérile selon l'une des revendications 7 à 14, **caractérisé en ce que** l'un (36) des deux supports (36, 40) présente une ouverture d'échange de gaz (34) en liaison fluidique avec l'environnement (60) du récipient stérile (10), et **en ce que** les protubérances annulaires (38) dudit un (36) des deux supports (36, 40), entourent de manière concentrique l'ouverture d'échange de gaz (34).

16. Récipient stérile selon l'une des revendications 7 à 15, **caractérisé en ce que** le deuxième support (40) est monté sur le récipient stérile (10) de manière mobile par rapport au premier support (36).

17. Récipient stérile selon l'une des revendications 7 à 16, **caractérisé en ce qu'**il est prévu au moins une butée (48) pour prédéfinir une distance d'espacement minimale (52) entre le premier et le deuxième support (36, 40).

18. Récipient stérile selon la revendication 17, **caractérisé en ce que** le deuxième support (40) porte ladite au moins une butée (48).

19. Récipient stérile selon la revendication 17 ou la revendication 18, **caractérisé en ce que** ladite au moins une butée (48) est réalisée sous la forme d'une protubérance (48), et **en ce qu'**une hauteur (52) de la protubérance (48) correspond à la distance d'espacement minimale (52) entre le premier et le deuxième support (36, 40).

20. Récipient stérile selon l'une des revendications précédentes, **caractérisé en ce que** la barrière stérile (36, 38, 40, 44) et/ou l'ouverture d'échange de gaz (34) sont d'une configuration de forme circulaire ou sensiblement circulaire.

21. Récipient stérile selon l'une des revendications 4 à 20, **caractérisé en ce que** le support de maintien (66) comprend au moins un élément de maintien (72) pour maintenir et/ou soutenir au moins une partie (40, 44) de la barrière stérile (36, 38, 40, 44).

22. Récipient stérile selon la revendication 21, **caractérisé en ce que** ledit au moins un élément de maintien (72) est monté de manière mobile sur le récipient stérile (10).

23. Récipient stérile selon l'une des revendications 21 ou 22, **caractérisé en ce que** ledit au moins un élément de maintien (72) est maintenu sous précontrainte sur le récipient stérile (10), de sorte que le récipient stérile (10), pour une différence de pression, qui est inférieure à la différence de pression minimale, prend la position stérile.

24. Récipient stérile selon l'une des revendications 21 à 23, **caractérisé en ce que** la barrière stérile (36, 38, 40, 44) présente au moins un secteur de maintien, **en ce que** le support de maintien (66) comprend au moins un élément de maintien, et **en ce que** ledit au moins un secteur de maintien s'appuie sur ledit au moins un élément de maintien (84).

25. Récipient stérile selon la revendication 24, **caractérisé en ce que** ledit au moins un élément de maintien (72) présente un bras de maintien (84) qui surmonte le secteur de maintien et est agencé de manière à s'étendre parallèlement ou sensiblement parallèlement à une paroi (32) du récipient stérile (10), qui porte la barrière stérile (36, 38, 40, 44).

26. Récipient stérile selon l'une des revendications 21 à 25, **caractérisé en ce que** ledit au moins un élément de maintien (72) s'étend sur un secteur angulaire dans la direction périphérique de la barrière stérile (36, 38, 40, 44).

27. Récipient stérile selon la revendication 26, **caractérisé en ce que** le secteur angulaire présente des valeurs de 10° à 50°, en particulier de 20°.

28. Récipient stérile selon l'une des revendications 21 à 27, **caractérisé en ce que** sont prévus au moins deux éléments de maintien (72), et **en ce que** lesdits au moins deux éléments de maintien (72) sont agencés de manière symétrique autour de la barrière stérile (36, 38, 40, 44).

29. Récipient stérile selon l'une des revendications précédentes, **caractérisé en ce que** la barrière stérile (36, 38, 40, 44) est agencée sur le couvercle (14).

30. Récipient stérile selon l'une des revendications précédentes, **caractérisé en ce qu'**il est prévu au moins une butée (80) pour limiter une section de passage de gaz (92) maximale du parcours d'écoulement stérile (58).

31. Récipient stérile selon l'une des revendications précédentes, **caractérisé en ce que** le couvercle (14) et/ou la barrière stérile (36, 38, 40, 44) est ou sont fabriqués en une matière plastique, en particulier en polyétheréthercétone (PEEK) ou polyphénylsulfone (PPSU).

32. Récipient stérile selon l'une des revendications précédentes, **caractérisé en ce que** dans la position en pression, la pression régnant dans l'environnement du récipient stérile (10) dépasse la pression régnant dans le compartiment de réception (16) au moins d'une valeur égale à la différence de pression minimale.
